# EUROPEAN PATENT APPLICATION

(11) **EP 1 683 777 A1**
(43) Date of publication of application: **26.07.2006**
(21) Application number: 06001354.7
(22) Date of filing: 23.01.2006
(51) Int. Cl.: C07C 1/26

(54) **A process for producing an aralkyl compound, a dehalogenation method of an aralkyl halide compound and a method for recovering an aralkyl compound**

(30) Priority: 25.01.2005 JP 2005016678
(71) Applicant: Sumitomo Chemical Company, Limited, Tokyo 104-8260 (JP)
(72) Inventor: Yamada, Seiji, Kurashiki-shi Okayama (JP); Kishimoto, Kiyomi, Toyonaka-shi Osaka (JP)
(74) Representative: Vossius & Partner

(57) **Abstract**

The present invention provides a process for producing an aralkyl compound which comprises catalytically hydrogenating an aralkyl halide compound in a two-layer solvent system comprising water and water-insoluble organic solvent, a dehalogenation method of aralkyl halide compounds by subjecting to catalytic reduction in a two-layer solvent system comprising water and a water-insoluble organic solvent, and
a method for recovering aralkyl compound from waste occurred in production of aralkyl halide compound by halogenation of aralkyl compound using the dehalogenation method.

## Description

The present invention relates to a process for producing an aralkyl compound and a dehalogenation method of aralkyl halide compounds. The invention further relates to a method for recovering an aralkyl compound.

Aralkyl monohalide compounds are useful compounds for producing aralkyl derivatives in the field of fine chemicals such as medicine and agricultural chemicals. For example, 4'-bromomethyl-2-cyanobiphenyl is a useful chemical as an intermediate for depressors.
The aralkyl monohalide compounds are generally obtained by reacting aralkyl compounds with halogenating agents such as chlorinating agents, brominating agents and the like. In this reaction, however, dihalides and un-reacted aralkyl compounds other than monohalides, the objects, are present in the reaction mixture. This presence requires separating and purifying operations such as distillation and recrystallization to separate the objective aralkyl monohalide compounds. After such operation, the residues after distillation, filtrates after filtration have been usually disposed without recycling.
Disposing highly expensive aralkyl compounds is economically disadvantageous, and also needs additional cost for treatment to avoid the increase of environmental load. Because of these reasons, reuse of aralkyl halide has been desired. Several methods therefore have been proposed, for example, JPH06-00184008-A, JPH10-251213-A, J. Am. Chem. Soc., 74, 127(1952), and the like. Because they require high hydrogen pressure, require a base, require cumbersome solvent replacement operations, or the like, the conventional methods above are not necessarily satisfactory as industrial or economical methods.

Objects of the present invention are to provide a process for producing an aralkyl compound and a dehalogenation method of aralkyl halide compound without defects the conventional methods have.
Another object of the present invention is to provide a simple method for recovering aralkyl compound from waste by-produced in production of aralkyl halide compound by halogenation of aralkyl compound.
These and other objects will be apparent from the following descriptions.
The present invention relates to the followings:
<1> A process for producing an aralkyl compound which comprises catalytically hydrogenating an aralkyl halide compound in a two-layer solvent system comprising water and water-insoluble organic solvent.
<2> The process according to <1>, wherein the organic solvent is at least one selected from the group consisting of chlorobenzene, ethyl acetate and dichlorobenzene.
<3> The process according to <1> or <2>, wherein the hydrogenation is conducted with hydrogen and in the presence of supported palladium catalyst or supported platinum catalyst.
<4> The process according to any one of <1> to <3>, wherein the hydrogenation is conducted in the presence of activated carbon.
<5> The process according to any one of <1> to <4>, wherein the amount of water is 0.5 to 10 parts by weight per one part by weight of the aralkyl halide compound.
<6> The process according to any one of <1> to <5>; wherein the aralkyl halide compound is at least one selected from the group consisting of 4'-bromomethyl-2-cyanobiphenyl, 4'-dibromomethyl-2-cyanobiphenyl, 4'-bromomethyl-2-(t-butyloxycarbonyl)biphenyl and 4'-dibromomethyl-2-(t-butyloxycarbonyl)biphenyl.
<7> A dehalogenation method of aralkyl halide compounds by subjecting to catalytic reduction in a two-layer solvent system comprising water and a water-insoluble organic solvent.
<8> The method according to <7>, wherein the organic solvent is at least one selected from the group consisting of chlorobenzene, ethyl acetate and dichlorobenzene.
<9> The method according to <7> or <8>, wherein the hydrogenation is conducted with hydrogen and in the presence of supported palladium catalyst or supported platinum catalyst.
<10> The method according to any one of <7> to <9>, wherein the hydrogenation is conducted in the presence of activated carbon.
<11> The method according to any one of <7> to <10>, wherein the amount of water is 0.5 to 10 parts by weight per one part by weight of the aralkyl halide compound.
<12> The method according to any one of <7> to <11>, wherein the aralkyl halide compound is at least one selected from the group consisting of 4'-bromomethyl-2-cyanobiphenyl, 4'-dibromomethyl-2-cyanobiphenyl, 4'-bromomethyl-2-(t-butyloxycarbonyl)biphenyl and 4'-dibromomethyl-2-(t-butyloxycarbonyl)biphenyl.
<13> A method for recovering aralkyl compound from waste occurred in production of aralkyl halide compound by halogenation of aralkyl compound, which subjects the waste to catalytic reduction in a two-layer solvent system comprising water and a water-insoluble organic solvent.
<14> The method according to <13>, wherein the waste is filtrate obtained after crystallization and filtration of aralkyl halide compound.
<15> The method according to <13> or <14>, wherein the waste is distillation residue obtained after distillation of aralkyl halide compound.
<16> The method according to any one of <13> to <15>, wherein the production of aralkyl halide compound is carried out by halogenating aralkyl compound with halogen atom.
<17> The method according to any one of <13> to <16>, wherein the organic solvent is at least one selected from the group consisting of chlorobenzene, ethyl acetate and dichlorobenzene.
<18> The method according to any one of <13> to <17>, wherein the hydrogenation is conducted with hydrogen and in the presence of supported palladium catalyst or supported platinum catalyst.
<19> The method according to any one of <13> to <18>, wherein the hydrogenation is conducted in the presence of activated carbon.
<20> The method according to any one of <13> to <19>, wherein the amount of water is 0.5 to 10 parts by weight per one part by weight of the aralkyl halide compound.
<21> The method according to any one of <13> to <20>, wherein the aralkyl halide compound is at least one selected from the group consisting of 4'-bromomethyl-2-cyanobiphenyl, 4'-dibromomethyl-2-cyanobiphenyl, 4'-bromomethyl-2-(t-butyloxycarbonyl)biphenyl and 4'-dibromomethyl-2-(t-butyloxycarbonyl)biphenyl.

The aralkyl compound can be produced by catalytically hydrogenating an aralkyl halide compound in a two-layer solvent system comprising water and water-insoluble organic solvent. The aralkyl halide compound can be dehalogenated by subjecting to catalytic hydrogenation in a two-layer solvent system comprising water and a water-insoluble organic solvent.
Examples of the aralkyl halide compounds used for the present invention include benzyl halides, benzal halides, naphthyl halides, pyridylmethyl halides and quinolyl halides. The aralkyl halide compound may be aralkyl monohalide compound, aralkyl dihalide compound or a mixture thereof. These aralkyl halide compounds may have substituent(s).
Examples of the substituent include phenyl group; alkoxycarbonyl group having 2 to 12 carbon atoms such as methoxycarbonyl group, ethoxycarbonyl group and propoxycarbonyl group; halogen atom such as fluorine atom, chlorine atom and bromine atom; tetrazolyl group; and perfluoroalkyl group such as trifluoromethyl group.
The phenyl group may further have substituent group. Examples thereof include phenyl group; alkoxycarbonyl group having 2 to 12 carbon atoms such as methoxycarbonyl group, ethoxycarbonyl group and propoxycarbonyl group; halogen atom such as fluorine atom, chlorine atom and bromine atom; tetrazolyl group; and perfluoroalkyl group such as trifluoromethyl group.
Examples of the halide in the aralkyl halide compound include fluorine atom, chlorine atom, bromine atom and iodine atom. Chlorine atom and bromine atom are preferred from the viewpoint of dehalogenation.
Examples of such aralkyl halide compounds include 4'-bromomethyl-2-cyanobiphenyl, 4'-dibromomethyl-2-cyanobiphenyl, 4'-bromomethyl-2-(t-butyloxycarbonyl)biphenyl and 4'-dibromomethyl-2-(t-butyloxycarbonyl)biphenyl.
The solvent used in the present invention is two-phase solvent system comprising water and water-insoluble solvent.
The water-insoluble organic solvent is not particularly limited as long as a solvent usable to catalytic hydrogenation, and the solubility of the organic solvent in water at normal temperature is usually less than 20 % by volume, preferably less than 10 % by volume. Examples of the water-insoluble solvents include halogenated hydrocarbons such as monochlorobenzene and dichlorobenzene; esters such as ethyl acetate; and aromatic hydrocarbons such as toluene.
From the viewpoints of reactivity and volume efficiency, the amount of the water-insoluble organic solvent used is usually 1 to 50 parts by weight per 1 part by weight of the aralkyl halide compound, and preferably 5 to 30 parts by weight.
From the viewpoints of volume efficiency and prevention of side reaction, the amount of the water is usually 0.5 to 10 parts by weight per 1 part by weight of the aralkyl halide compound and preferably 1 to 6 parts by weight.
When it is expressed by (the amount by weight of water)/(the amount by weight of the water-insoluble organic solvent), the ratio of water and water-insoluble organic solvent is usually 1/20 or higher and preferably 1/10 to 1/1.
Examples of the catalyst include supported palladium catalysts such as Pd/C and supported platinum catalysts such as Pt/C, and Pd/C is economically preferred. The amount of the metal (Pt, Pd, etc.) contained is, not particularly limited, generally 1 to 20 % by weight, and preferably 5 to 10 % by weight in the catalyst.
From the viewpoints of reactivity and economical efficiency, the amount of the catalyst used is generally 0.01 to 2 % by weight in terms of metal of the catalyst based on the total amount of the aralkyl halide compound, and preferably 0.05 to 1 part by weight.
From the viewpoints of reaction velocity and prevention of side reaction such as dealkylation, the temperature of catalytic hydrogenation is usually 0 °C or higher, preferably 10 to 90 °C, and more preferably 50 to 80 °C.
From the viewpoints of reaction velocity, safety and prevention of side reaction, pressure for the hydrogenation is from an atmospheric pressure to pressure of 1000 mmH₂O in terms of hydrogen pressure, and preferably from an atmospheric pressure to 600 mmH₂O.
From the viewpoint of smooth proceeding of the reaction, activated carbon can preferably be present in the catalytic hydrogenation of the present invention.
As the activated carbon, commercially available activated carbon and carbon for decolorization may be used.
The amount of the activated carbon is generally 1 to 30 parts by weight, and preferably 5 to 20 parts by weight per 100 parts by weight of the aralkyl halide compound.
Completion of the catalytic hydrogenation can be checked by the cease of hydrogen absorption. This may also be checked by disappearance of the aralkyl halide compound observed by HPLC measurement.
Post-treatment methods are not particularly limited, which may use general methods applied to isolate aralkyl compounds. For example, filtering the catalyst, subjecting the filtrate to phase separation, and then further washing the separated organic layer with aqueous alkali solution, water and the like, and subjecting to concentration; thereafter, the aralkyl compound can be isolated by crystallization, distillation or the like.

By employing the dehalogenation method of aralkyl halide compound described above, aralkyl compound can be recovered from waste produced in production of aralkyl halide compound by halogenation of aralkyl compound.
The aralkyl compound can be recovered by subjecting the waste to catalytic reduction in two-layer solvent system comprising water and a water-insoluble organic solvent.
After the reaction (halogenation) of aralkyl compound, aralkyl halide compound produced is usually subjected to after-treatment for isolation and purification. In the after-treatment, the aralkyl halide having relatively higher melting point is crystallized and filtered to collect. After the crystallization and filtration, filtrate containing aralkyl halide compound is obtained. When the aralkyl halide has relatively lower melting point, the aralkyl halide compound is purified by distillation. After the distillation, distillation residue containing the aralkyl halide compound is obtained.
In the present invention, waste means liquid or solid by-produced and remained in the course of isolation or purification of aralkyl halide compound and containing the aralkyl halide compound. Examples thereof include the filtrate above, the distillation residue above, and the like.
The waste contains at least one of aralkyl monohalide compound and aralkyl dihalide compound, and may contain the raw material, unreacted aralkyl compound. The content of the aralkyl halide compound in the waste may vary depending of the method of isolation or purification, conditions thereof, or the like, and is not limited.
The waste can be subjected to catalytic hydrogenation in the same manner as disclosed in the foregoing dehalogenation method.
Post-treatment processes for recovering aralkyl compound by dehalogenation may be used by general recovery methods applied to aralkyl compound. For example, after filtering the catalyst, subjecting the filtrate to phase separation, further washing the separated organic layer with aqueous alkali solution or salt solution and concentrating the organic layer, the aralkyl compounds can be recovered by crystallization, distillation or the like.

The halogenation reaction of aralkyl compound can be carried out by any known methods utilizing halogenating agents such as halogen atoms. For example, the method described in WO 03/106406 can be applied.
Specifically, the halogenation method is achieved by, for example, reacting 4'-methyl-2-cyanobiphenyl with bromine in a solvent in the presence of a radical initiator and an oxidant. As to the order of addition of the reagents, it is preferable that bromine or a solution thereof be added to a mixture of 4'-methyl-2-cyanobiphenyl, a radical initiator and an oxidant charged in a solvent in advance, from the aspect of operability. It is also possible to simultaneously add bromine, a radical initiator or a solution thereof. For smooth progress of the reaction, moreover, the reaction mixture is preferably placed under stirring. The amount of bromine to be used is usually 0.4-0.7 mol, preferably 0.45-0.60 mol, more preferably 0.52-0.58 mol, per 1 mol of 4'-methyl-2-cyanobiphenyl.
As the radical initiator, azobis-compound, peroxide and the like are used. Specifically, the azobis-compound is exemplified by 2,2'-azobis(4-methoxy-2,4-dimethylvaleronitrile), 2,2'-azobisisobutyronitrile, 2,2'-azobis(2-methylbutyronitrile) and 2,2'-azobis(2,4-dimethylvaleronitrile); and peroxide is exemplified by dibenzoyl peroxide, di-t-butyl peroxide and the like. Preferred are 2,2'-azobis(2-methylbutyronitrile) and 2,2'-azobis(2,4-dimethylvaleronitrile), and particularly preferred is 2,2'-azobis(2-methylbutyronitrile). The amount of a radical initiator to be used is usually 0.1 to 10 mol %, preferably 1 to 4 mol %, relative to 4'-methyl-2-cyanobiphenyl, which is a starting material.
The oxidant is exemplified by oxidants whose handling is comparatively safe, such as bromates (e.g., sodium bromate, potassium bromate and the like); and chlorates (e.g., sodium chlorate, potassium chlorate and the like), with preference given to sodium bromate. The amount of an oxidant to be used is an amount theoretically necessary for regenerating bromine from by-produced hydrogen bromide or a slightly excess amount thereof, which is 9-20 mol %, preferably 12-17 mol %, relative to 4'-methyl-2-cyanobiphenyl, which is a starting material.
The solvent to be used in the present invention is exemplified by halogenated hydrocarbon, alkane having 5 to 7 carbon atoms, aliphatic ester and the like. Specific examples thereof include methylene chloride, ethylene dichloride, carbon tetrachloride, monochlorobenzene, o-dichlorobenzene, bromobenzene, hexane, heptane, cyclohexane, methyl acetate, ethyl acetate, methyl propionate, ethyl propionate and the like. Of these, preferred are monochlorobenzene and ethyl acetate. The amount of the solvent to be used is generally 0.5 to 20 parts by weight, preferably 1 to 20 parts by weight, more preferably 1 to 15 parts by weight, most preferably 3 to 15 parts by weight, per 1 part by weight of 4'-methyl-2-cyanobiphenyl, which is a starting material.
In the halogenation reaction, the reaction system preferably contains water. The presence of water dramatically increases the effect of stirring, and makes the progress of the reaction smooth. The content of water is 0.01 to 50% (w/w), preferably 0.05 to 30% (w/w), relative to the solvent to be used. Even when the content of water is a catalytic amount (about 0.01 to 2.0% (w/w), preferably 0.05 to 0.5% (w/w), relative to the solvent to be used), the reaction proceeds sufficiently.
While the reaction temperature varies depending on the radical initiator and the like, it is generally 50-100 °C, preferably 50-85°C, more preferably 60-85°C, most preferably 60-70°C. The radical can be also produced by photoirradiation of radical initiator. In this case, a mercury lamp and the like can be used. The reaction time is also determined appropriately depending on the above-mentioned various reaction conditions (e.g., about 3-10 hr).
The 4'-bromomethyl-2-cyanobiphenyl obtained by the above-mentioned reaction is isolated and purified from a reaction mixture. The method therefor may be, but not limited to, a conventional method including removing inorganic salts by filtration and the like, distilling away the solvent as necessary, and recrystallizing from a different suitable solvent.

The recovered aralkyl compound can be re-used for the production of aralkyl halide compound by halogenation.

The invention is explained more specifically with reference to examples, however, the invention is not limited thereto in any way.
HPCL measurement conditions
Column: SUMIPAX ODS A-212
Column temperature: 40°C
Carrier medium:
   A: methyl alcohol
   B : 0.1 % aqueous acetic acid solution
Carrier medium concentration: 60 % to 100 %
Flow rate of Carrier medium: 1.0 ml/minute
Retention time RRT (Relative Retention Time) .1.0 4'-bromomethyl-2-cyanobiphenyl RRT 1.1 4'-methyl-2-cyanobiphenyl
RRT 1.2 4'-dibromomethyl-2-cyanobiphenyl

### Production Example

Chlorobenzene (652.5 g) was added with 4'-methyl-2-cyanobiphenyl (416.2 g, 2.251 mole) and sodium bromate (57.41 g, 0.38 mole) dissolved in water (109 g), and the mixture was warmed at 75 to 80°C.
A solution of 2,2'-azobis(2-methylbutyronitrile) (8.31 g, 0.043 mole) dissolved in monochlorobenzene (87 g) was dropped together with bromine (217.5 g, 1.116 mole) into the mixed solution above at 70 to 80°C over 5 hours, then leaving it for 1 hour with maintaining its temperature. The solution was set still at about 80°C and then subjected to phase separation. The organic layer was added with 217.5 g of water, washed at about 80°C and then subjected to phase separation.
The separated solution was concentrated under a reduced pressure to eliminate 91.4 g of chlorobenzene. The residual solution was added with 304.5 g of chlorobenzene to filter at 75 to 80°C by a filter pre-coated with 11 g of diatomaceous earth. Washing with 208.8 g of chlorobenzene, then the filtrate was cooled down after confirming no precipitation of crystal observed therein. The filtrate was added with 450 mg of seed crystal at the temperature thereof of about 50°C, and stirred at 55°C for 6 hours. Then, this solution was cooled down in the rate of 10°C/hour, followed by stirring at the temperature thereof of 30 to 35°C for 5 hours and at the temperature thereof of 0 to -5°C for 10 hours, and then being subjected to filtration.
By rinsing the crystal with heptane, 404.6 g of 4'-bromomethyl-2-cyanobiphenyl was obtained in the yield of 69 %.

### Example

In a 500ml four-neck flask, 300g of filtrate before heptane rinsing of the above described Production Example (according to determination by HPLC-ES method; 4'-bromomethyl-2-cyanobiphenyl 4.75 %, 4'-dibromomethyl-2-cyanobiphenyl 3.93 % and 4'-methyl-2-cyanobiphenyl 5.43 %: according to determination by HPCL area percentage method; 4'-bromomethyl-2-cyanobiphenyl 28.4 %, 4'-dibromomethyl-2-cyanobiphenyl 23.5 % and 4'-methyl-2-cyanobiphenyl 32.5 %), 85 g of water, 2.52 g of activated carbon and 2.52 g of 5 % Pt/C (50 % wet) were added, followed by replacing the atmosphere thereof with nitrogen, and then the mixture was subjected to hydrogenation reaction at 60 to 70°C under 100 to 400 mmH₂O pressure of hydrogen. The HPCL area percentage values after completion of the reaction were 0.31 % of 4'-bromomethyl-2-cyanobiphenyl, non-detected 4'-dibromomethyl-2-cyanobiphenyl and 82.7 % of 4'-methyl-2-cyanobiphenyl.
Solid, mainly the catalyst and activated carbon, was separated by filtration, followed by separation of water layer from the filtrate to obtain an organic layer. The organic layer was washed with aqueous caustic soda solution and then with salt solution, followed by phase separation. Chlorobenzene in the organic phase was evaporated under a reduced pressure, followed by subjecting the residual solution to reduced-pressure distillation to obtain 32 g of 4'-methyl-2-cyanobiphenyl. Its dehalogenation yield was 95 % and purity was 98 %.

By brominating the 4'-methyl-2-cyanobiphenyl by the method described in Production Example and the like, 4'-bromomethyl-2-cyanobiphenyl, can be obtained.

According to the process and method of the invention, aralkyl halide compound can be dehalogenated to produce aralkyl compound by a simple operation of being subjected to catalytic hydrogenation in a two-phase solvent system comprising water and a water-insoluble organic solvent; and furthermore, aralkyl compound can be recovered by dehalogenation from a filtrate and a distillation residue containing the aralkyl halide compounds which are by-produced in the course of producing aralkyl halide compound.

## Claims

1. A process for producing an aralkyl compound which comprises catalytically hydrogenating an aralkyl halide compound in a two-layer solvent system comprising water and water-insoluble organic solvent.

2. A dehalogenation method of aralkyl halide compounds by subjecting to catalytic reduction in a two-layer solvent system comprising water and a water-insoluble organic solvent.

3. A method for recovering aralkyl compound from waste occurred in production of aralkyl halide compound by halogeriation of aralkyl compound, which subjects the waste to catalytic reduction in a two-layer solvent system comprising water and a water-insoluble organic solvent.

4. The method according to Claim 3, wherein the waste is filtrate obtained after crystallization and filtration of aralkyl halide compound.

5. The method according to Claim 3, wherein the waste is distillation residue obtained after distillation of aralkyl halide compound.

6. The method according to Claim 3, wherein the production of aralkyl halide compound is carried out by halogenating aralkyl compound with halogen atom.

7. The process according to claim 1 or the method according to Claim 2 or 3, wherein the organic solvent is at least one selected from the group consisting of chlorobenzene, ethyl acetate and dichlorobenzene.

8. The process according to claim 1 or the method according to Claim 2 or 3, wherein the hydrogenation is conducted with hydrogen and in the presence of supported palladium catalyst or supported platinum catalyst.

9. The process according to claim 1 or the method according to Claim 2 or 3, wherein the hydrogenation is conducted in the presence of activated carbon.

10. The process according to claim 1 or the method according to Claim 2 or 3, wherein the amount of water is 0.5 to 10 parts by weight per one part by weight of the aralkyl halide compound.

11. The process according to claim 1 or the method according to Claim 2 or 3, wherein the aralkyl halide compound is at least one selected from the group consisting of 4'-bromomethyl-2-cyanobiphenyl, 4'-dibromomethyl-2-cyanobiphenyl, 4'-bromomethyl-2-(t-butyloxycarbonyl)biphenyl and 4'-dibromomethyl-2-(t-butyloxycarbonyl)biphenyl.
